**Europäisches Patentamt**

(19) **European Patent Office**

**Office européen des brevets**

(11) Publication number : **0 233 061 B1**

(12) # EUROPEAN PATENT SPECIFICATION

(45) Date of publication of patent specification :
**12.06.91 Bulletin 91/24**

(51) Int. Cl.⁵ : **G01N 33/569,** G01N 33/546, G01N 33/555

(21) Application number : **87301017.7**

(22) Date of filing : **05.02.87**

(54) **Reagent for detecting antibody to viral antigen.**

(30) Priority : **06.02.86 JP 24626/86**

(43) Date of publication of application :
**19.08.87 Bulletin 87/34**

(45) Publication of the grant of the patent :
**12.06.91 Bulletin 91/24**

(84) Designated Contracting States :
**CH DE FR GB IT LI NL SE**

(56) References cited :
**EP-A- 0 105 465**
**EP-A- 0 106 495**
**EP-A- 0 135 352**
**EP-A- 0 206 842**
**GB-A- 1 362 977**
**CHEMICAL ABSTRACTS, vol. 107, no. 1, 06 July 1987, Columbus, OH (US); X.LAN et al., p. 509, no. 5408b**
**Macmillan Dictionary of Genetics and Cell Biology; pp. 191-195**

(73) Proprietor : **FUJIREBIO KABUSHIKI KAISHA also trading as FUJIREBIO INC.**
**6-7, Shimoochiai 4-chome Shinjuku-ku Tokyo 161 (JP)**

(72) Inventor : **Ikeda, Mikio**
**7-25-23, Sunagawa-cho**
**Tachikawa-shi Tokyo (JP)**
Inventor : **Mizukoshi, Mikio**
**1-8-6, Tsurugaoka Ohimachi**
**Iruma-gun Saitama-ken (JP)**

(74) Representative : **Silverman, Warren et al**
**HASELTINE LAKE & CO. Hazlitt House 28 Southampton Buildings Chancery Lane London WC2A 1AT (GB)**

## Description

This invention relates to a reagent for detecting an antibody to a virus relating to acquired immunodeficiency syndrome (AIDS) and which is considered to cause AIDS disease.

Acquired immunodeficiency syndrome is a new disease which was identified in 1981 (M.S. Gottlib et al., N. Eng. J. Med., 305, 1425 (1981)). Subsequently, a retrovirus named lymphadenopathy associated virus (LAV) was found in France to be present in lymphadenopathy syndrome patients (F. Barrs-Sinnoussi et al., Science, 220, 868 (1983)), and retroviruses named respectively human T-lymphotropic virus type III (HTLV-III) (M. Popovic et al., Science, 224, 497 (1984) and AIDS-associated retrovirus (ARV) (J.A.Levy et al., Science, 225, 840 (1984)) were subsequently found, in turn, to be present in AIDS or pre-AIDS patients in the U.S.A. Recently, the nucleic acid sequences of genes of these viruses LAV, HTLV-III and ARV, were completely analysed, and it was elucidated that these are variants of the same virus (L. Ratner et al., Nature, 313, 277 (1985), R. Sanchez-Pescaoor et al., Science, 227, 484 (1985), S. Wain-Hobson et al., Cell, 40, 9 (1985)). These variants have come to be known commonly as human immunodeficiency virus (HIV) (J. Coffin et al., Science, 232, 697 (1986). HIV infection occurs through contact between humans or as a result of a blood transfusion. It has also become known that an antibody specifically reacting with HIV is generated in the blood of a person infected with HIV. Accordingly, infection with HIV may be established by detecting this antibody.

Indirect immunofluorescence assay (IF), enzyme-linked immunosorbent assay (ELISA), radioimmunoassay (RIA) and Western Blot technique (US-A-4,520,113), Japenese Patent KOKAI Nos. 60-67859 and 60-256378) have been developed for detecting this specific antibody to HIV.

In the IF method, HIV-producing cells are immobilised on a slide glass, and a sample serum is spotted on it to react with the cells. The unreacted portion of the serum is removed by washing, and then, a fluorescein conjugated antihuman antibody is allowed to react further. After the reaction, unreacted fluorescent antibody is removed by washing, and the presence of an antibody to HIV is seen from the presence of specific fluorescence on the surface of the cells by observation using a fluorescence microscope. In the ELISA method, the characteristic antigenic component of HIV is coated on 96-well microtitre plate wells and a sample serum is allowed to react in a similar manner as the above IF. After washing, instead of the above-mentioned fluorescent antibody, an enzyme-labelled antihuman immunoglobulin is allowed to react, and unreacted enzyme-labelled antibody is washed off. A substrate specific to the enzyme is added, and the presence of an antibody to HIV is judged by the degree of colouring. The underlying principle behind the RI method is the same as that in the ELISA method except that radioisotope is employed as the labelling substance instead of enzyme. In the Western Blot technique, a protein antigenic to HIV is developed by electrophoresis, and then, transferred to a nitrocellulose membrane. The antigenic protein is allowed to react with a sample serum, and the reaction between a specific protein of HIV and a specific antibody is detected as a protein band.

However, with both the IF method and the Western Blot method, biohazard facilities are necessary because HIV or HIV-producing cells were employed. Moreover, their analysing capacities are small, and they are not suitable as a clinical test. In contrast, the ELISA method does not require biohazard facilities, and the anti-HIV antibody may be detected in a high sensitivity. However, it is a complicated process and special equipment is necessary. In addition, a long time is necessary for carrying out the measurement. Moreover, non-specific reaction has also been reported (T. Kurimura, "Rinsho to Uirusu", 14, 50 (1986)), and confidence in the results has not been sufficiently high.

An object of this invention is to provide a reagent capable of detecting an antibody to HIV in a simple operation with it being possible to have a high confidence in the detected results.

According to the present invention, there is provided a reagent for detecting an antibody to human immunodeficiency virus (HIV) by a passive particle agglutination method, which comprises carrier particles sensitized with HIV antigenic component.

This invention also provides a method of detecting an antibody to HIV, wherein test serum is subjected to passive particle agglutination carried out by using the reagent of the present invention.

Finally, this invention provides a reagent kit for detecting antibody to HIV which comprises, as separately packaged elements, carrier particles sensitized with HIV antigenic component, unsensitized like carrier particles, positive serum and a diluent for suspending the sensitized carrier particles.

The antigenic component of HIV is obtained by culturing HIV-producing cells. After the cultivation, the HIV produced is separated from culture supernatant and the recovered virus is solubilized by means of a surfactant. The HIV containing material is then centrifuged, and supernatant is recovered to obtain the antigenic component of HIV.

Any type of cell capable of producing HIV are usable as the HIV-producing cells. For example, since it is known that cells having T4 antigen which is a surface antigen of human T cell can be infected with HIV (D. Klatzmann et al., Science, 225, 59 (1984)), HIV-producing cells can be obtained by infecting the cells having this surface antigen with HIV. Furthermore, it is also known that when some known cell lines such as H9 cell line (M. Popovic et al., Science, 224, 497 (1984)), CEM cell line (R. Cheingsong-Popovic et al., Lancet, 11, 477 (1984), HUT 78 cell line (J.A. Levy et al., Science, 225, 840 (1984)) and Molt-4 cell line (S. Harada et al., Jpn. J. Cancer Res., 76, 432 (1985)) are infected with HIV, these cells become HIV-producing cells capable of subculture for a long period. These cells are also usable in the practice of this invention. Among them, Molt-4 cell line is the most preferable.

Cultivation of the HIV-producing cells can be carried out by following any desired cell culturing procedure. Any suitable culture medium may be used, examples of preferred culture media being RPMI 1640 medium and Eagle's minimum essential medium to which foetal calf serum (FCS)or calf serum has been added. Culture conditions may also be conventional and generally, culturing is carried out at pH 6.4 to 7.6 at 36 to 38°C. During culturing, culture medium is exchanged at an interval of 3 to 5 days, and, as a result, the desired cells multiply well to produce HIV in the culture liquid.

HIV may be separated from the culture supernatant by a conventional method such as centrifuging. Density-gradient ultracentrifugation is particularly preferable for this purpose because HIV can be obtained in high purity.

The antigenic component of HIV can be obtained by solublizing the above HIV by means of a surfactant capable of dissolving virus particles in a conventional manner. Nonionic surfactants such as Nonidet P-40® and Triton X-100® and anionic surfactants such as sodium dodecyl sulphate and sodium deoxycholate are usable as the surfactant.

The solubilization may be carried out, for example, by adding a buffer solution containing 0.01 to 2% by weight of the surfactant to the above HIV and stirring at 0 to 25°C for 30 minutes to 24 hours. The solublized solution thus obtained is centrifuged, and its supernatant is employed as the antigenic component of HIV.

The carrier particles on which the antigenic component of HIV is sensitized may be conventional for passive particle agglutination. Examples of such particles include erythrocytes of warm-blooded animals such as human, sheep and chicken, as well as gelatin particles (US-A-4,416,813). Gelatin particles are preferable for use in the practice of this invention because they show little non-specific reaction and the gelatin is easily modified to add properties necessary for the use of the particles.

The sensitization of the antigenic component of HIV may be carried out by one of a variety of known methods for sensitizing an antigen on carrier particles. Such methods include the use of a coupling reagent, such as tannic acid, glutaraldehyde, bis-diazotized benzidine, tolylene diisocyanate, difluoro-dinitrobenzene, carbodiimides, quinones, and chromium chloride, and the adsorption method. The sensitization is preferably carried out under slightly acidic conditions such as pH 5 to 7 as shown below.

As an indication of the effect of pH on sensitization, gelatin particles of the type employed in section (2) of Example 1 which follows were sensitized with the antigenic component of HIV prepared in section (1) of Example 1 in a similar manner to that described in section (2) of Example 1 but varying pH as shown in Table 1. The gelatin particles sensitized with the antigenic component of HIV thus obtained were tested in conventional manner, and the results are shown in Table 1.

T a b l e   1

| Sensitization pH | Titre Test | Control Test |
|---|---|---|
| 4.0 | − | − |
| 5.0 | + | − |
| 6.0 | + | − |
| 7.0 | + | − |
| 8.0 | Cannot be determined | + |

−: Agglutination does not occur

+: Agglutination occurs

In the above table, by "Titre Test" is meant the reaction between the antigen-sensitized gelatin particles and positive serum in the antibody to HIV, and by "Control Test" is meant reaction between the antigen-sensitized gelatin particles and a diluent for serum.

The carrier particles sensitized with the antigenic component of HIV are usually lyophilized, and supplied with a reconstituting solution, a diluent, a standard serum, unsensitized carrier particles and an absorbing solution, and supplied for use. The diluent is preferably slightly alkaline such as at pH 7 to 9 to optimize conditions for agglutination, as shown below.

The antigen-sensitizied gelatin particles of Table 1 prepared at pH 6.0 were tested using diluents of various pH values in a conventional manner, and the results are shown in Table 2.

## T a b l e  2

| pH of Diluent | Titre Test | Control Test |
|---|---|---|
| 6.0 | Cannot be determined | + |
| 7.0 | + | − |
| 8.0 | + | − |
| 9.0 | + | − |
| 10.0 | − | − |

In the above table, the meanings of −, +, Titre Test and Control Test are the same as in Table 1.

The measurement of the antibody to an antibody to HIV may be carried out by following the conventional method of utilizing indirect passive hemagglutination. For example, each sample serum is placed in wells of a micro titre plate, and each requisite dilution series is prepared. The suspension of antigen-sensitized particles is added to each well, and stirred. Then, each mixture is allowed to stand for 1 to 3 hours in its well, and the agglutination pattern of these sensitized particles is judged by observation.

By using the reagent of the invention, many sample sera can be analysed accurately and rapidly in a simple operation. Non-specific reaction does not occur. With the IF and ELISA methods, several operations, including washing operations, are required. In contrast, when using the reagent of the present invention, a diluent is added to each well, and then the mixture in the well is merely allowed to stand for a predetermined time. The result may be judged by the naked eye, and no special apparatus for determination is necessary. The antigenic component used in the reagent of the invention is safe, because the infectious characteristics of the HIV are removed by the treatment with a surfactant.

The following Examples illustrate this invention :

## EXAMPLE 1

(1) Preparation of HIV Antigenic Component

Molt-4 cells were infected with HIV to produce a HIV-producing cell line. This cell line was seeded into a RPMI-1640 culture medium containing 10% Foetal bovine serum, and cultured at 37°C for 4 days under an atmosphere of sterile air containing 5% $CO_2$. 20 litre of the culture solution were centrifuged at 3,000 rpm for 10 minutes to remove cells, and supernatant was collected. This supernatant was layered over 0 to 50% sucrose density gradient which had been previously prepared in a continuous zonal rotor, and ultracentrifugation was carried out at 30,000 rpm while the supernatant was circulated.

Virus fractions were layered again over a 20 to 55% sucrose continuous density gradient which had previously been prepared in a centrifuge tube, and ultracentrifugation was carried out using a swing-type rotor at 25,000 rpm for 4 hours. Virus fractions were collected, and centrifuged at 45,000 rpm for 60 minutes to precipitate virus particles. A phosphate buffered saline solution containing 0.5% by weight Nonidet P-40 was added to the virus particles which became suspended therein. The suspension was stirred for 30 minutes in an ice bath to dissolve the virus particles, and then was centrifuged at 13,000 rpm for 30 minutes. The supernatant thus obtained was to be employed as the antigenic component of HIV.

4

(2) Preparation of Sensitized Particles

Gelatin particles were prepared according to the method described in Example 1 of US-A-4,416,813. The gelatin particles were suspended in 0.15 M phosphate buffered saline solution (PBS) (pH 6.0) containing 5 ppm of tannic acid in a concentration of 2.5% by weight, and allowed to warm at 37°C for 10 minutes. The particles were collected by centrifuging, and washed 3 times with a saline solution. The washed particles were suspended in 0.15 M PBS (pH 6.0) in a concentration of 5% by weight.

In a separate operation, the antigenic HIV composition prepared in Section (1) was diluted 50 times with 0.15 M PBS (pH 6.0), and this diluted antigen was mixed with equal volume of the above suspension of tannic acid-treated particles. The mixture obtained was allowed to warm at 37°C for 40 minutes, and the particles were thereby sensitized with the antigenic composition of HIV. The sensitized particles were washed three times with a saline solution by centrifugation, and suspended in a suspension solution which was prepared by dissolving 1.920 g disodium hydrogenphosphate dodecahydrate, 0.291 g potassium dihydrogen phosphate, 0.438 g sodium chloride 0.25 g gum arabic, 1.5 g glycine, 0.01 g thimerosal and 1.0 ml normal rabbit serum in a purified water so that the total volume of the solution was 100 ml. The suspension obtained was lyophilized.

(3) Preparation of Diluent

1.920 g disodium hydrogen phosphate dodecahydrate, 0.291 g potassium dihydrogen phospate, 0.438 g sodium chloride, 0.25 g gum arabic and 0.15 g sodium azide were dissolved in purified water so that the total volume of the solution was 100 ml. This solution was available for use as a diluent.

(4) Preparation of Kit

The antigen-sensitized gelatin particles were packaged with the above diluent, the lyophilized unsensitized gelatin particles produced in section (2), a lyophilized positive serum material for control purposes, a solution for suspending the sensitized gelatin particles and a diluent for sampale serum to form a reagent kit for detecting antibody to HIV.

EXAMPLE 2

Sheep erythrocytes were treated with formalin in conventional manner, and suspended in 0.15 M PBS (pH 6.0) in a concentration of 5 v/v%, and an equal volume of 0.001 w/v% tannic acid 0.15 M PBS (ph 6.0) solution was added. The mixture was allowed to react at 37°C for 15 minutes with occasional stirring. The erythrocytes were washed with a saline solution, and suspended in 0.15 M PBS (pH 6.0) in a concentration of 5 v/v% to obtain a suspension of tannic acid-treated erythrocytes.

The antigenic component prepared in stage (1) of Example 1 was diluted 50 times with 0.15 M PBS (pH 6.0), and an equal volume of this diluted antigen was added to the above suspension of tannic acid-treated erythrocytes. the mixture was allowed to warm at 37°C for 30 minutes. the erythrocytes were centrifuged, and washed twice with 0.15 M PBS (pH 7.2). The washed erythrocytes were placed in the suspension solution of stage 2 of Example 1 and lyophilized.

1.920 g disodium hydrogen phosphate dodecahydrate, 0.291 g potassium dihydrogen phospate, 0.438 g sodium chloride, 0.25 g gum arabic, 0.15 sodium azide, 1.0 ml normal rabbit serum, 2.0 ml sheep erythrocyte stroma and 1.0 ml bovine erythrocyte stroma were dissolved in purified water so that total volume of the solution obtained for use as diluent was 100 ml.

The above lyophilized antigen sensitized erythrocyte material was packaged with unsensitized carrier eythrocytes, positive serum for control and the above diluent to form a reagent kit for detecting antibody to HIV.

EXAMPLE 3

Passive particle agglutination tests were carried out by using the reagent kits obtained in Examples 1 and 2 in tests carried out on various sample sera.

The results are shown in Table 3.

T a b l e   3

|  | | Invention | | | | Conventional | |
| Serum Sample | Number of Samples | Ex. 1 | | Ex. 2 | | IF | |
|  |  | Posi-tive | Nega-tive | Posi-tive | Nega-tive | Posi-tive | Nega-tive |
| AIDS Patients | 32 | 32 | 0 | 32 | 0 | 32 | 0 |
| ARC Patients | 15 | 15 | 0 | 15 | 0 | 15 | 0 |
| Healthy Homo-sexuals | 5 | 0 | 5 | 0 | 5 | 0 | 5 |
| ATLA Positive Sera | 24 | 0 | 24 | 0 | 24 | 0 | 24 |

## Claims

1. A reagent for detecting an antibody to human immunodeficiency virus (HIV) by a passive particle agglutination method, which comprises carrier particles sensitized with HIV antigenic component.

2. The reagent of claim 1, wherein HIV whose antigenic component sensitizes the carrier particles has been produced by culturing a cell line selected from H9 cell line, CEM cell line, HUT 78 cell line and Molt-4 cell line, which has been infected with HIV.

3. The reagent of claim 1 or 2, wherein HIV whose antigenic component sensitizes the carrier particles has been separated by density gradient ultracentrifugation from supernatant from the culture solution in which it has been produced.

4. The reagent of any preceding claim, wherein said antigenic component has been produced from HIV by dissolving HIV virus particles in a surfactant capable of dissolving virus particles.

5. The reagent of any preceding claim, wherein said carrier particles are gelatin particles.

6. The reagent of any preceding claim, wherein sensitization of said carrier particles with said antigenic component has been carried out at pH 5 to 7.

7. A reagent kit for detecting antibody to HIV which comprises as separately packaged elements carrier particles sensitized with HIV antigenic component, unsensitized like carrier particles, positive serum and a diluent for suspending the sensitized carrier particles.

8. A reagent kit as claimed in claim 7, wherein both said particle types and/or the serum are lyophilized and the kit additionally comprises a reconstituting solution for said lyophilized particles and/or a diluent for said serum.

9. A method of detecting an antibody to HIV, wherein test serum is subjected to passive particle agglutination carried out by using a reagent as claimed in any one of claims 1 to 6.

10. The method of claim 9, wherein the test serum is diluted with a diluent at pH 7 to 9.

## Ansprüche

1. Reagenz zum Nachweis eines Antikörpers gegen das humane immundefiziente Virus (HIV) durch eine passive Teilchenagglutinationsmethode, die die Trägerteilchen, die mit dem HIV-Antigenbestandteil sensibilisiert wurden, enthält.

2. Reagenz nach Anspruch 1, bei dem das HIV, dessen Antigenkomponente, das die Trägerteilchen sensibilisiert hat, durch Kultivierung einer Zellinie, ausgewählt aus H9-Zellinie, CEM-Zellinie, HUT-78-Zellinie und Molt-4-Zellinie, die mit HIV infiziert wurden, hergestellt wurde.

3. Reagenz nach Anspruch 1 oder 2, bei dem das HIV, dessen Antigenkomponente, das die Trägerteilchen sensibilisiert hat, durch Dichtegradientenultrazentrifugation aus dem Überstand der Kulturlösungen, in denen es produziert wurde, aufgetrennt wurde.

4. Reagenz nach einem der vorhergehenden Ansprüche, bei dem die besagte Antigenkomponente aus HIV, durch lösen von HIV-Virus-Partikeln in einem Lösungsmittel, das fähig ist, die Viruspartikel zu lösen, hergestellt wurde.

5. Reagenz nach einem der vorhergehenden Ansprüche, bei dem die Trägerteilchen Gelatineteilchen sind.

6. Reagenz nach einem der vorhergehenden Ansprüche, bei dem die Sensibilisierung der Trägerteilchen mit der Antigenkomponente bei einem pH von 5-7 durchgeführt wurde.

7. Reagenzsatz zum Antikörpernachweis von HIV, der als einzeln verpackte Bestandteile, Trägerteilchen, die mit der HIV-Antigenkomponente sensibilisiert wurden, sowie unsensibilisierte Trägerteilchen, Positivserum und ein Lösungsmittel zum Suspensieren der sensibilisierten Trägerteilchen enthält.

8. Reagenzsatz nach Anspruch 7, bei dem beide Teilchentypen und/oder das Serum lyophylisiert sind, und der Reagenzsatz zusätzlich eine Rekonstitutionslösung für die lyophylisierten Teilchen und/oder ein Lösungsmittel für das Serum enthält.

9. Verfahren zum Nachweis eines Antikörpers gegen HIV, bei dem das Testserum einer passiven Teilchenaglutination unterzogen wurde, die unter Verwendung eines Reagenz nach einem der Ansprüche 1-6 durchgeführt wurde.

10. Verfahren nach Anspruch 9, bei dem das Testserum in einem Lösungsmittel bei einem pH von 7-9 gelöst wurde.

## Revendications

1. Réactif pour la détection d'un anticorps dirigé contre le virus de l'immunodéficience humaine (HIV) par une méthode d'agglutination passive de particules, qui comprend des particules de support sensibilisées avec un composant antigénique du HIV.

2. Réactif selon la revendication 1, dans lequel le HIV dont le composant antigénique sensibilise les particules de support a été produit par culture d'une lignée cellulaire, choisie parmi la lignée cellulaire H9, la lignée cellulaire CEM, la lignée cellulaire HUT 78 et la lignée cellulaire Molt-4, qui a été infectée par le HIV.

3. Réactif selon la revendication 1 ou 2, dans lequel le HIV dont le composant antigénique sensibilise les particules de support a été séparé par ultracentrifugation en gradient de densité, à partir du surnageant de la solution de culture dans laquelle il a été produit.

4. Réactif selon l'une quelconque des revendications précédentes, dans lequel ledit composant antigénique a été produit à partir de HIV par dissolution de particules de virus HIV dans un agent tensioactif capable de dissoudre les particules de virus.

5. Réactif selon l'une quelconque des revendications précédentes, dans lequel lesdites particules de support sont des particules de gélatine.

6. Réactif selon l'une quelconque des revendications précédentes, dans lequel la sensibilisation desdites particules de support par ledit composant antigénique a été effectuée à pH 5-7.

7. Trousse de réactif pour la détection d'anticorps dirigé contre le HIV, qui comprend/en tant qu'éléments conditionnés séparément, des particules de support sensibilisées par le composant antigénique du HIV, des particules de support semblables non sensibilisées, un sérum positif et un diluant pour la mise en suspension des particules de support sensibilisées.

8. Trousse de réactif selon la revendication 7, dans laquelle les deux types de particules et/ou le sérum sont lyophilisés et la trousse comprend en outre une solution de reconstitution pour lesdites particules lyophilisées et/ou un diluant pour ledit sérum.

9. Procédé de détection d'un anticorps dirigé contre le HIV, dans lequel on soumet un sérum d'essai à une agglutination passive de particules effectuée au moyen d'un réactif selon l'une quelconque des revendications 1 à 6.

10. Procédé selon la revendication 9, dans lequel le sérum d'essai est dilué avec un diluant à pH 7-9.